# EUROPEAN PATENT APPLICATION

(11) **EP 4 378 462 A1**
(43) Date of publication of application: **05.06.2024**
(21) Application number: 22849591.7
(22) Date of filing: 28.07.2022
(51) Int. Cl.: A61K 31/4965, A61P 11/00, A61P 31/14, C07D 241/24

(54) **THERAPEUTIC AGENT FOR COVID-19**

(30) Priority: 30.07.2021 JP 2021125337
(71) Applicant: FUJIFILM Toyama Chemical Co., Ltd., Chuo-ku Tokyo 104-0031 (JP)
(72) Inventor: SAKURAI, Tsutomu, Tokyo 104-0032 (JP); YAMATAKE, Takahiro, Tokyo 104-0032 (JP)
(74) Representative: Wächtershäuser & Hartz Patentanwaltspartnerschaft mbB
(86) International application number: PCT/JP2022/029139
(87) International publication number: WO 2023/008530

(57) **Abstract**

The present invention provides a therapeutic agent for novel coronavirus infection comprising 6-fluoro-3-hydroxy-2-pyrazinecarboxamide or a salt thereof as an active ingredient, which is administered to novel coronavirus infection patients testing negative for either one or both of IgG antibody against novel coronavirus and IgA antibody against novel coronavirus.

## Description

### TECHNICAL FIELD

The present invention relates to a therapeutic agent for novel coronavirus infection comprising 6-fluoro-3-hydroxy-2-pyrazinecarboxamide (nonproprietary name, favipiravir; hereinafter referred to as Compound A) or a salt thereof as an active ingredient.

### BACKGROUND ART

The novel coronavirus (SARS-CoV-2) first reported in China at the end of 2019 (Non-patent Literature 1) is an RNA virus, which belongs to the family coronaviridae, order nidovirales (Non-patent Literature 2). Infection with this virus causes acute respiratory symptoms, such as pyrexia, cough, and dyspnea, and pneumonia occurs when the disease progresses.

Compound A has been reported as a substance that has potential to be effective for the treatment of novel coronavirus infection (Non-patent Literature 3). A Japanese phase III clinical study of Compound A in COVID-19 patients with non-serious pneumonia has demonstrated with a statistically significant difference that time from the start of administration of the study drug to a time point where body temperature, percutaneous arterial blood oxygen saturation (SpOz), and chest image findings are improved and SARS-CoV-2 is tested "negative conversion," the primary endpoint, is shorter by approximately 3 days in the Compound A group than in the control group, indicating that administration of Compound A to COVID-19 patients with non-serious pneumonia improves their symptoms early (Non-patent Literature 4).

In general, the effectiveness of a therapeutic agent depends on the patient's characteristics. However, details in the relationship between the therapeutic effect of Compound A and the patient's characteristics are unknown for novel coronavirus infection.

### PRIOR ART DOCUMENT

### NON PATENT DOCUMENT

Non Patent Document 1: Wang C, Horby PW, Hayden FG, Gao GF. A novel coronavirus outbreak of global health concern. Lancet. 2020;395:470-3.
Non Patent Document 2 : Coronaviridae Study Group of the International Committee on Taxonomy of Viruses. The species Severe acute respiratory syndrome-related coronavirus: classifying 2019-nCoV and naming it SARS-CoV-2. Nat Microbiol. 2020 Mar 2. PubMed PMID: 32123347.
Non Patent Document 3: ACS Central Science (2020), 6(3), 315-331
Non Patent Document 4 : "The primary endpoint was achieved in a Japanese clinical phase III study in patients with novel coronavirus infection" (News release dated 23 September 2020). [Internet]. Fujifilm Co., Ltd. Available from: https://www.fuj ifilm.com/jp/ja/news/list/5451

### SUMMARY OF INVENTION

### TECHNICAL PROBLEM

No treatment methods have been established for novel coronavirus infection, and there is a need to develop a therapeutic agent for novel coronavirus infection early. An object of the present invention is to provide a therapeutic agent for novel coronavirus infection and a method for treating novel coronavirus infection.

### SOLUTION TO PROBLEM

Under the above-described circumstances, the present inventors have found that Compound A can be used more effectively in patients with novel coronavirus infection who have a lower titer of an antibody against novel coronavirus, and accomplished the present invention.

According to the present invention, the following inventions are provided.
[1] A therapeutic agent for novel coronavirus infection comprising 6-fluoro-3-hydroxy-2-pyrazinecarboxamide or a salt thereof as an active ingredient, which is administered to novel coronavirus infection patients testing negative for either one or both of IgG antibody against novel coronavirus and IgA antibody against novel coronavirus.
[2] The therapeutic agent for coronavirus infection according to [1], which is administered to patients testing negative for both the IgG antibody against novel coronavirus and the IgA antibody against novel coronavirus.
[3] The therapeutic agent for coronavirus infection according to [1] or [2], wherein the patients are patients with non-serious pneumonia.
[4] The therapeutic agent for novel coronavirus infection according to any one of [1] to [3], wherein the patients are patients who meet all the following criteria (1) and (2):
   (1) Patients who have a pulmonary lesion on a chest image; and
   (2) Patients who have a fever of 37.5°C or higher.
[5] The therapeutic agent for novel coronavirus infection according to any of [1] to [4], wherein 6-fluoro-3-hydroxy-2-pyrazinecarboxamide is administered at 1,000 to 2,400 mg twice a day on Day 1 and at 400 to 1,200 mg twice a day on Day 2 and thereafter.
[6] The therapeutic agent for novel coronavirus infection according to any of [1] to [4], wherein 6-fluoro-3-hydroxy-2-pyrazinecarboxamide is administered at 1,800 mg of twice a day on Day 1 and at 800 mg twice a day on Day 2 and thereafter.
[7] The therapeutic agent for novel coronavirus infection according to any of [1] to [6], which is administered for 14 days.

According to the present invention, the following inventions are also provided:
(a) A pharmaceutical composition comprising Compound A or a salt thereof for the treatment of novel coronavirus infection, the pharmaceutical composition administered to patients testing negative for either one or both of IgG antibody against novel coronavirus and IgA antibody against novel coronavirus;
(b) Compound A or a salt thereof for the treatment of novel coronavirus infection, the Compound A or a salt thereof administered to patients testing negative for either one or both of IgG antibody against novel coronavirus and IgA antibody against novel coronavirus.
(c) A method for treating novel coronavirus infection comprising administration of Compound A or a salt thereof, the method comprising administration of Compound A or a salt thereof to patients testing negative for either one or both of IgG antibody against novel coronavirus and IgA antibody against novel coronavirus.
(d) Use of Compound A or a salt thereof to manufacture a therapeutic agent for novel coronavirus infection administered to patients testing negative for either one or both of IgG antibody against novel coronavirus and IgA antibody against novel coronavirus.

### ADVANTAGEOUS EFFECTS OF INVENTION

Novel coronavirus infection can be treated by administering Compound A or a salt thereof to novel coronavirus infection patients testing negative for either one or both of IgG antibody against novel coronavirus and IgA antibody against novel coronavirus.

### DESCRIPTION OF EMBODIMENTS

[Figure 1] Patient deposition.
[Figure 2] Figure 2 shows time to improvement of COVID-19 in the analysis population (mITT).
[Figure 3] Figure 3 shows time to improvement of COVID-19 in a subgroup (testing negative for IgG antibody and IgA antibody).

### [Description of Embodiments]

The present invention will be described in detail below.

In this specification, each term has the following meaning unless otherwise specified.

In this specification, a range of numerical values represented using "-" means a range including the numerical values before and after "-" as the minimum value and the maximum value, respectively.

Compound A means 6-fluoro-3-hydroxy-2-pyrazinecarboxamide.

Examples of a salt of Compound A include commonly known salts in basic groups such as an amino group and acidic groups such as a hydroxyl group or a carboxyl group.

Examples of the salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salts in acidic groups include salts with alkali metals such as sodium and potassium; salts with alkaline earth metals such as calcium and magnesium; ammonium salts; and salts with nitrogen-containing organic basic compounds such as trimethylamine, triethylamine, tributylamine, pyridine, N,N dimethylaniline, *N*-methylpiperidine, *N-*methylmorpholine, diethylamine, dicyclohexylamine, procaine, dibenzylamine, *N*-benzyl-β-phenethylamine, 1-efenamine, *N*,*N*-dibenzylethylenediamine, and meglumine.

Among the above-mentioned salts, examples of preferred salts include pharmacologically acceptable salts, and examples of more preferred salts include salts with sodium or meglumine.

If Compound A or a salt thereof has isomers (e.g., optical isomers, geometrical isomers, and tautomers), the present invention encompasses all these isomers, as well as hydrates, solvates, and all crystal forms.

The term "coronavirus" used in this specification means an RNA virus belonging to the family coronaviridae, order nidovirales. The term "novel coronavirus" means a newly identified and reported novel coronavirus among coronaviruses, and examples thereof include SARS-CoV-2 reported at the end of 2019. Infection caused by novel coronavirus is novel coronavirus infection. The infectious disease caused by SARS-CoV-2 is also called COVID-19. The term "novel coronavirus" naturally encompasses mutants and variants of the known novel coronavirus. Examples of variants of SARS-CoV-2 include VOC-202012/01, 501Y.V2, 501Y.V3, GRY (formerly GR/501Y.V1), G/478K.V1, GH/452R.V1, GR/484K.V2, G/484K.V3, GR/1092K.V1, GH/253G.V1, G/452R.V3, GR/452Q.V1, and GR.

Treatment means relieving or improving one or more symptoms caused by a specific disease that affects a target patient, as well as delaying the progression of the disease. In an embodiment of the present invention, for example, treatment means delaying progression of the disease or relieving or improving symptoms, such as pyrexia, cough, and pneumonia. In an embodiment of the present invention, treatment means improving body temperature, percutaneous arterial blood oxygen saturation (SpO₂), and chest image findings or achieving recovery to novel coronavirus negative conversion. The term "progression of the disease" used herein means that a patient with novel coronavirus infection falls into a condition requiring administration of oxygen in some way or dies.

From a viewpoint that a relative therapeutic effect is higher in patients to whom Compound A or a salt thereof is administered, as compared with patients to whom Compound A or a salt thereof is administered, patients with a low titer of an antibody against novel coronavirus are preferred. Specifically, for example, patients testing negative for either one or both of IgG antibody against novel coronavirus and IgA antibody against novel coronavirus are preferred, and patients testing negative for both IgG antibody against novel coronavirus and IgA antibody against novel coronavirus are more preferred. IgG antibody and IgA antibody mean immunoglobulin G and immunoglobulin A, respectively. IgG antibody against novel coronavirus and IgA antibody against novel coronavirus mean IgG antibody and IgA antibody produced by infection with novel coronavirus or vaccination against novel coronavirus. For example, testing negative for IgG antibody against novel coronavirus means that an antibody test shows presence of IgG antibody below the detection limit in serum.

Among patients with a low titer of an antibody against novel coronavirus and patients testing negative for either one or both of IgG antibody against novel coronavirus and IgA antibody against novel coronavirus, patients who further have a complication of non-serious pneumonia are more preferred. The term "non-serious pneumonia" used herein means pneumonia that does not accompany hypoxemia requiring oxygen therapy.

Compound A or a salt thereof used in the present invention can be manufactured by using methods known per se or by using them in combination appropriately. For example, Compound A or a salt thereof can be manufactured by the methods described in International Publication No. WO 00/10569. Compound A has a tautomer 6-fluoro-3-oxo-3,4-dihydro-2-pyrazinecarboxamide.

Compound A or a salt thereof used in the present invention can be formulated as pharmaceutical formulations such as oral agents (e.g., tablets, capsules, powders, granules, subtilized granules, pills, suspensions, emulsions, solutions, syrups), injections, eye drops, nasal agents, and percutaneous agents by adding various pharmaceutical additives, such as excipients, binders, disintegrating agents, disintegration-inhibiting agents, hardening/adhesion-preventing agents, lubricants, carriers for absorption/adsorption, solvents, extenders, isotonizing agents, dissolving aids, emulsifiers, suspending agents, thickeners, coating agents, absorption-promoting agents, gelatinization/coagulation-promoting agents, photostabilizers, preservatives, desiccants, emulsification/suspension/dispersion-stabilizing agents, discoloration-preventing agents, deoxygenation/antioxidation agents, flavoring/odor-improving agents, coloring agents, foaming agents, defoaming agents, soothing agents, antistatic agents, and buffers/pH modifiers. Preparations administered to patients with novel coronavirus infection are preferably oral agents or injections, more preferably oral agents, yet more preferably tablets.

The above-mentioned pharmaceutical agents are formulated by usual methods.

The method of administering Compound A is not particularly limited but is suitably determined depending on the form of the formulations, the patient's age, sex, and other conditions, and the severity of the patient's symptoms.

The half maximal effective concentration (EC50) of Compound A against SARS-CoV-2 was 61.88 µM in a study using Vero E6 cells (Wang M, Cao R, Zhang L, Yang X, Liu J, Xu M, et al. Remdesivir and chloroquine effectively inhibit the recently emerged novel coronavirus (2019-nCoV) in vitro. Cell Res. 2020;30:269-71). This concentration is equivalent to 9.72 µg/mL. However, when the above documents were published, the effects of this treatment administered to patients actually infected with this virus could not be predicted.

The dose of Compound A is suitably selected depending on the dosing regimen, the patient's age and sex, disease form, and other conditions. For example, Compound A can be administered to adults at 10 to 5,000 mg or preferably 200 to 2,400 mg once or divided into several doses a day. Compound A can be administered as multiple doses or a single dose until the intended therapeutic effect is achieved. The administration is typically monitored, and Compound A can be administered repeatedly, if necessary.

In the present invention, Compound A can be administered to adults at 1,000 to 2,400 mg BID on Day 1 and at 400 to 1,200 mg BID on Day 2 and thereafter. It is preferable that Compound A is administered to adults preferably at 1,600 mg twice a day on Day 1 and at 600 mg twice a day on Day 2 and thereafter, that Compound A is administered to adults preferably at 1,800 mg twice a day on Day 1 and at 800 mg twice a day on Day 2 and thereafter, and that Compound A is administered to adults preferably at 1,800 mg twice a day on Day 1 and at 1,000 mg twice a day on Day 2 and thereafter. It is more preferable that Compound A can be administered to adults at 1,800 mg twice a day on Day 1 and at 800 mg twice a day on Day 2 and thereafter.

For an interval of BID administration, the second dose is administered preferably after an interval of at least 4 hours, more preferably 6 hours or more from the first dose.

The duration of administration is suitably determined depending on changes overtime in symptoms, and for example, can be selected from up to 5 days, 6 days, 7 days, 8 days, 9 days, 10 days, 11 days, 12 days, 13 days, 14 days, 15 days, 16 days, 17 days, 18 days, 19 days, 20 days, 21 days, and 22 days. The durations of up to 10 days, 13 days, 14 days, and 22 days are preferred, and the duration of up to 13 days and 14 days are more preferred.

In the present invention, the administration of Compound A or a salt thereof can include administration of a concomitant drug and/or performing a concomitant therapy. Specifically, the standard of care for pneumonia can be included. The "standard of care for pneumonia" used herein is treatment described in Section "3 Treatment" of the "Guidance for Clinical Practice of Novel Coronavirus Infection (COVID-19) First Edition" published by the Novel Coronavirus Infection Measures Promotion Division, the Ministry of Health, Labour and Welfare, on March 17, 2020 in Japan. Specifically, the standard of care for pneumonia means that "if pneumonia is clinically diagnosed in a patient suspected of infection, it is recommended to start an antimicrobial drug empirically without waiting for the result of pathogen diagnosis. Caution should be exercised not to infuse excess fluid, and oxygen inhalation should be initiated, if necessary. (omission) Even after a definite diagnosis of COVID-19, appropriate antibacterial drugs should be administered if concurrent bacterial infection is suspected" or treatment similar thereto. Additionally, oxygen therapy can be included. Further, interferon-alpha formulations, ribavirin, or drugs having an antiviral effect against novel coronavirus can also be included. Examples of the drugs having an antiviral effect against novel coronavirus include the following drugs a) to e):
a) Hydroxychloroquine sulfate, chloroquine phosphate;
b) Lopinavir/ritonavir combination;
c) Ciclesonide;
d) Nafamostat mesilate; and
e) Camostat mesilate.

The present invention will be described using a clinical trial as an example, but the present invention is not limited to the example.

### Test Example 1: An adaptive, single-blind, randomized, multicenter, comparative study to evaluate the efficacy and safety of Compound A in COVID-19 patients with non-serious pneumonia

### [Methodology]

1. Study design
   1.1 Type of study
      Confirmatory study (phase 3)
   1.2 Study design
      Adaptive, single-blind, randomized, multi-center, comparative study
   1.3 Group composition and administration methods
      [Compound A group]
      Standard of care + Compound A 1,800 mg BID for 1 day + 800 mg BID for 13 days (maximum) [Control group]
      Standard of care + Compound A placebo 9 tablets BID for 1 day + 4 tablets BID for 13 days (maximum)
   1.4 Endpoints
      1.4.1 Primary efficacy endpoint
         Time to improvement in body temperature, SpOz and chest imaging, and recovery to negative conversion of SARS-CoV-2
      1.4.2 Secondary efficacy endpoints
         (1) Changes overtime in clinical status on the 7-point scale
         (2) Change in overtime in SARS-CoV-2 genome amount
         (3) Time to elimination of SARS-CoV-2
         (4) Durations of body temperature, SpO₂ and findings from chest imaging by symptom/finding
         (5) Changes overtime in clinical symptoms/findings
         (6) Change overtime in vital signs
         (7) Change overtime in the National Early Warning Score (NEWS)
         (8) Rate of improvement in findings from chest imaging on Days 4, 7, 10, 13, 16, 19, 22, 25, and 28
         (9) Proportion of patients who require supplemental oxygen therapy and the mean duration
         (10) Proportion of patients who require mechanical ventilation therapy and the mean duration
         (11) Changes overtime in white blood cell count, hemoglobin, platelet count, creatinine, blood sugar, total bilirubin, ALT and AST
      1.4.3 Safety endpoints
         (1) Adverse events
         (2) Laboratory test values
         (3) Vital signs
         (4) 12-Lead electrocardiogram
   1.5 Target number of patients
      As the number of patients enrolled: 96 patients (64 patients in the Compound A group, 32 patients in the control group).
2. Subject
   2.1 Subject
      COVID-19 patients with non-serious pneumonia
   2.2 Inclusion criteria
      (1) Age: 20 to 74 years old (at the time of consent)
      (2) Sex: Irrespective
      (3) Outpatient/hospitalization: Hospitalization
      (4) Patients who meet all the following criteria 1), 2) and 3) at the registration:
         1) Patients tested SARS-CoV-2 positive in the RT-PCR test or the like using respiratory samples such as nasopharyngeal swab, nasal aspirate and airway aspirate
         2) Patients with confirmed pulmonary lesions in chest images
         3) Patients with a fever of 37.5°C or higher
      (5) In female patients of childbearing potential, patients who are tested negative in the pregnancy test conducted before the start of administration
      (6) Patients who understand the content of this clinical trial and abele to submit written consent by himself/herself
   2.3 Exclusion criteria
      (1) Patients whose fever of 37.5°C or higher developed 10 days ago or before
      (2) Patients who used interferon alpha formulations and drugs whose anti-viral action against SARS-CoV-2 has been reported (hydrodxychloroquine sulfate, chloroquine phosphate, lopinavir/ritonavir combination drugs, ciclesonide, nafamostat mesilate, and camostat mesilate) within 9 days after development of a fever of 37.5°C or higher
      (3) Patients with an infection episode in question that is a relapse of SARS-CoV-2 infection or re-infection with SARS-CoV-2
      (4) Patients with SpO₂ below 95% without oxygen therapy
      (5) Patients suspected to have a complication of bacterial infection showing increases in procalcitonin values before the start of administration
      (6) Patients suspected to have complication of fungal infection showing abnormal (1→3)-β-D-glucan values before the start of administration
      (7) Patients suspected to have complication of congestive cardiac failure showing NT-proBNP values of 400 pg/mL or higher (or BNP values of 100 pg/mL or higher) before the start of administration
      (8) Patients with severe hepatic dysfunction equivalent to Grade C in the Child-Pugh classification
      (9) Patients with renal impairment requiring dialysis
      (10) Patient with consciousness disturbed including orientation disturbed
      (11) Patients who are pregnant or possibly pregnant
      (12) Female patients who do not agree to exercise contraception by birth control pill, intrauterine device and barrier method (diaphragm, condom) or combination of these methods from the start of administration of the study drug to 7 days after the end of administration
      (13) Male patients with partners who do not agree to exercise contraception using the contraceptive methods described in the above (12)
      (14) Patients who do not agree to use condoms from the start of administration of the study drug to 7 days after the end of administration
      (15) Patients with hereditary xanthinuria
      (16) Patients who have been diagnosed with hypouricemia (less than 1 mg/dL) or xanthine urinary calculus
      (17) Patients with a history of gout or on patients who are currently being treated for gout or hyperuricemia
      (18) Patients who are taking immunosuppressants
      (19) Patients who were administered Compound A
      (20) Patients who are deemed to be ineligible for the study by the principal investigator or a subinvestigator
3. Study drug
   Tablet A: One tablet contains 200 mg of Compound A (administered to the Compound A group)
   Tablet B: A tablet which is indistinguishable in appearance from Tablet A and does not contain Compound A (administered to the control group)
   Dosage form: Pale yellow, film-coated tablet
      Tablet A and Tablet B used in this study are manufactured by using the methods described in International Publication No. WO 2010/104170 or methods known per se or using these methods in combination appropriately.
4. Dosage, administration and treatment period
   4.1 Dosage and administration
      (1) Dose
         The standard of care plus the study drug at the following doses will be administered according to the treatment group: [Compound A group]
         Compound A 1,800 mg BID for 1 day + 800 mg BID for 13 days (maximum) [Control group]
         Compound A placebo 9 tablets BID for 1 day + 4 tablets BID for 13 days (maximum)
      (2) Administration method
         [Compound A group]
            Tablet A (containing 200 mg of Compound A) will be orally administered at 9 tablets BID on Day 1 and 4 tablets BID on Day 2 and thereafter for maximum 13 days.
         [Control group]
            Tablet B (placebo not containing Compound A) will be orally administered at 9 tablets BID on Day 1 and 4 tablets BID on Day 2 and thereafter for maximum 13 days.
            On Day 1, the second dose will be administered with at least 4 hour interval from administration of the first dose.
   4.2 Treatment period
      The treatment period is up to 14 days.
5. Criteria for evaluation
   5.1 Study procedures
      The clinical trial will be conducted in accordance with the following procedures:
      (1) Confirmation of inclusion/exclusion criteria, explanation of the clinical trial and obtaining informed consent, performing of observations and tests before the start of administration
      (2) Registration of the patient (enrollment to the study)
      (3) Assigning the patient to treatment
      (4) Prescription of the study drug and start of administration
      (5) Specified observations and tests, evaluations/determination
      (6) Follow-up investigation
   5.2 Evaluation criteria
      5.2.1 Efficacy evaluation criteria
         (1) Efficacy evaluation criteria
            1) Efficacy criteria for the primary endpoint
               Time from the start of administration of the study drug to a time point where SARS-CoV-2 is tested "negative conversion" 48 hours after "improvement" in body temperature, SpO₂ and findings from chest imaging will be evaluated as the primary endpoint.
               "Improvement" in body temperature, SpO₂ and findings from chest imaging is defined as a status where all parameters of body temperature, SpO₂ and findings from chest imaging meet and maintain the following criteria a) to c) and for at least 2 days (at least 48 hours as a guidance). Recovery to SARS-CoV-2 "negative conversion" is defined as a status where SARS-CoV-2 meets and maintains the following criterion d) for at least 12 hours.
                  a) Body temperature: 37.4°C or lower (observed values within 4 hours after the use of antipyretic analgesic drugs should not be used)
                  b) SpO₂: 96% or higher (without oxygen therapy)
                  c) Findings from chest imaging: improvement from the worst
                  d) SARS-CoV-2: Negative in the RT-PCR (qualitative) test
            2) Criteria for the secondary endpoints
               a) Time to elimination of SARS-CoV-2
                  Time from the start of administration of the study drug to recovery to SARS-CoV-2 "negative conversion" will be evaluated as time to elimination of SARS-CoV-2.
               b) Durations of body temperature, SpO₂ and findings from chest imaging by symptom/finding
                  Time from the start of administration of the study drug to a time point where body temperature, SpO₂ and findings from chest imaging meet and maintain the criteria a) to c) in the above 1) and r for at least 2 days (48 hours or longer as a guidance) will be evaluated as duration of each symptom/finding.
               c) NEWS classification
                  The score of each parameter of the NEWS classification from clinical symptoms/findings (disorientation) and vital signs (SpO₂, body temperature, blood pressure, pulse rate, and respiratory rate) and other records recorded by the principal investigator or a subinvestigator, and calculate a total score.
      5.2.2 Safety evaluation criteria
         The principal investigator or a subinvestigator will determine the severity of adverse events that have developed during the clinical trial and the causal relationships of these adverse events.

### [Results]

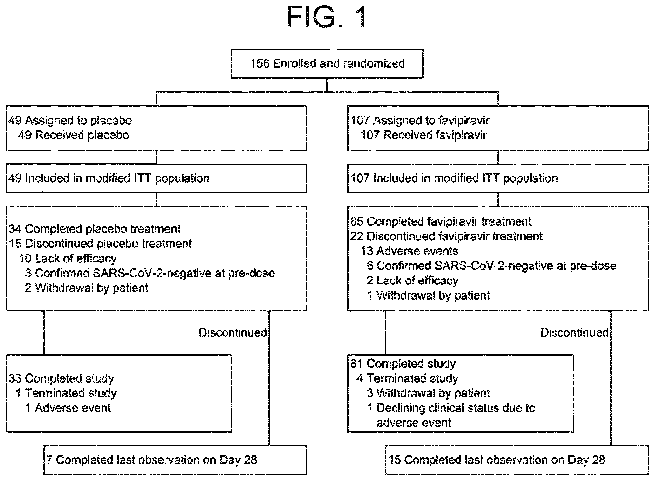

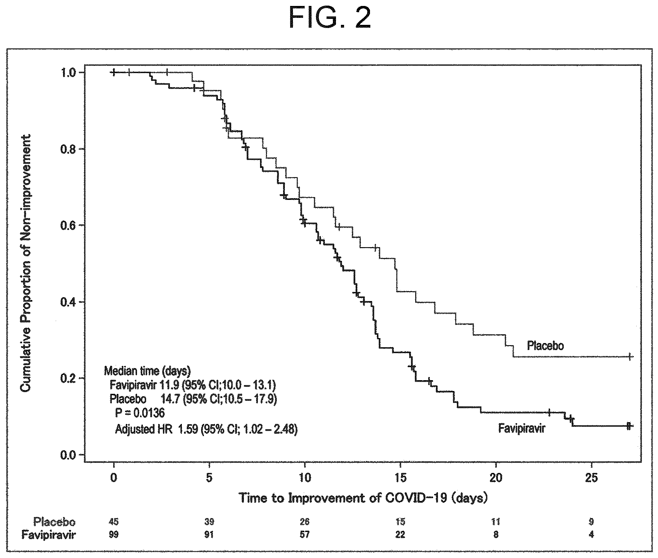

The efficacy and safety of Compound A were evaluated in 156 patients with COVID-19 (Compound A group, 107 patients; control group, 49 patients) in accordance with the method described in Test Example 1. The patient disposition is shown in Figure 1. To understand the condition of patients with COVID-19, improvement in body temperature, SpO₂ and chest imaging and recovery to negative conversion of SARS-CoV-2 were selected as an endpoint, and time from the initiation of study treatment to improvement of all body temperature, SpO₂ and chest imaging followed by confirmation of negative conversion of SARS-CoV-2 was evaluated. Patients who were discontinued from the study or study treatment because of inadequate response were censored on Day 28. The results are shown in Table 1 and Figure 2. The median time to improvement was shorter by approximately 3 days in the Compound A group than in the control group.

**[Table 1]**

| | | Control group (N = 49) | Compound A group ((N = 107) |
|---|---|---|---|
| Patients with improvement | | 28 | 81 |
| Censored patients | | 21 | 26 |
| Median time to improvement in 75% of patients (days) | | -- | 15.6 |
| | 95% confidence interval | -- | 13.7, 17.8 |
| Median time to improvement in 50% of patients (days) | | 14.7 | 11.9 |
| | 95% confidence interval | 10.5, 17.9 | 10.0, 13.1 |
| Median time to improvement in 25% of patients (days) | | 9.0 | 7.8 |
| | 95% confidence interval | 5.8, 11.6 | 6.7, 9.7 |
| P value^{a} | | 0.0136 | |
| Adjusted hazard ratio^{b} (Compound A vs. control) | | 1.593 | |
| | 95% confidence interval | 1.024, 2.479 | |

| | | | |
|---|---|---|---|
| a. Weighted log-rank statistics, based on Cui, Hung, and Wang (1989) b. Hazard ratio in a Cox proportional hazard model with time to improvement of COVID-19 as a response variable and sex, age, and treatment group as explanatory variables | | | |

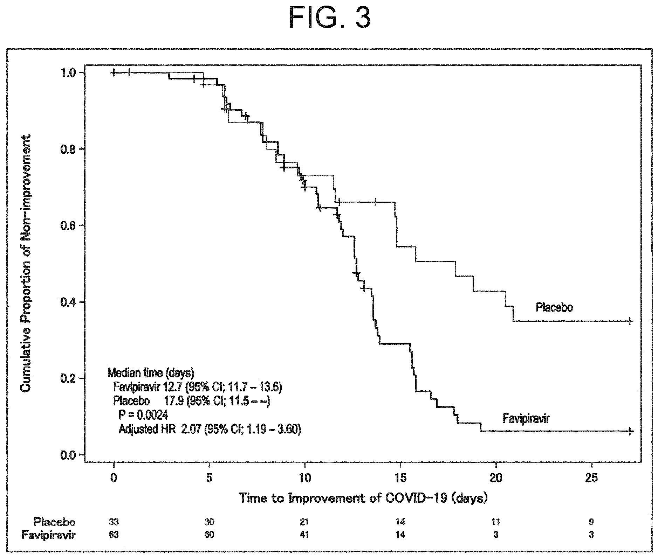

An IgG antibody against novel coronavirus and IgA antibody against novel coronavirus in patients were measured to perform a subgroup analysis. The results are shown in Figure 3. In the subgroup of patients testing negative for both IgG antibody and IgA antibody, the median time to improvement was shorter by approximately 5 days in the Compound A group than in the control group. The adjusted hazard ratio (Compound A vs. control) was 2.07 (95% confidence interval, 1.19-3.60). This result revealed that Compound A could be used more effectively in a patient with lower titers of antibodies against novel coronavirus.

## Claims

1. A therapeutic agent for novel coronavirus infection comprising 6-fluoro-3-hydroxy-2-pyrazinecarboxamide or a salt thereof as an active ingredient, which is administered to novel coronavirus infection patients testing negative for either one or both of IgG antibody against novel coronavirus and IgA antibody against novel coronavirus.

2. The therapeutic agent for coronavirus infection according to claim 1, which is administered to patients testing negative for both the IgG antibody against novel coronavirus and the IgA antibody against novel coronavirus.

3. The therapeutic agent for coronavirus infection according to claim 1 or 2, wherein the patients are patients with non-serious pneumonia.

4. The therapeutic agent for novel coronavirus infection according to any one of claims 1 to 3, wherein the patients are patients who meet both the following criteria (1) and (2):
(1) Patients who have a pulmonary lesion on a chest image; and
(2) Patients who have a fever of 37.5°C or higher.

5. The therapeutic agent for novel coronavirus infection according to any one of claims 1 to 4, wherein 6-fluoro-3-hydroxy-2-pyrazinecarboxamide is administered at 1,000 to 2,400 mg twice a day on Day 1 and at 400 to 1,200 mg twice a day on Day 2 and thereafter.

6. The therapeutic agent for novel coronavirus infection according to any one of claims 1 to 4, wherein 6-fluoro-3-hydroxy-2-pyrazinecarboxamide is administered at 1,800 mg twice a day on Day 1 and at 800 mg twice a day on Day 2 and thereafter.

7. The therapeutic agent for novel coronavirus infection according to any one of claims 1 to 6, which is administered for 14 days.
